# EUROPEAN PATENT APPLICATION

(11) **EP 3 971 302 A1**
(43) Date of publication of application: **23.03.2022**
(21) Application number: 20196462.4
(22) Date of filing: 16.09.2020
(51) Int. Cl.: C12Q 1/6827, C12Q 1/6858

(54) **METHOD FOR DETECTING 5-HYDROXYMETHYLATED CYTOSINE**

(71) Applicant: QIAGEN GmbH, 40724 Hilden (DE)
(72) Inventor: PEIST, Ralf, 40723 Hilden (DE); SINGER, Thorsten, 42699 Solingen (DE)
(74) Representative: Uexküll & Stolberg

(57) **Abstract**

The present invention generally relates to the field of detecting modified cytosine residues in a nucleic acid molecule. More specifically, the invention relates to an amplification-based method of indentifying and detecting 5-hydroxymethylated cytosine in a nucleic acid molecule. The method allows distinguishing 5-hydroxymethylated cytosine residues both from unmodified cytosine residues and 5-methylated cytosine residues. The method includes modification of the nucleic acid molecule with a bisulfite compound, preceded by oxidation or not) and the subsequent amplification of the nucleic acid molecule with at least one oligonucleotide (e.g. a 3`end matched or mismatched primer to C or U) that modulates the amplification efficiency depending on the presence of cytosine or uracil in at least one position of said nucleic acid molecule.

## Description

The present invention generally relates to the field of detecting modified cytosine residues in a nucleic acid molecule. More specifically, the invention relates to an amplification-based method of identifying and detecting 5-hydroxymethylated cytosine in a nucleic acid molecule. The method allows distinguishing 5-hydroxymethylated cytosine residues both from unmodified cytosine residues and 5-methylated cytosine residues. The method includes modification of the nucleic acid molecule with a bisulfite compound and the subsequent amplification of the nucleic acid molecule with at least one oligonucleotide that modulates the amplification efficiency depending on the presence of cytosine or uracil in at least one position of said nucleic acid molecule.

### BACKGROUND OF THE INVENTION

Epigenetics is a scientific field that describes the impact of heritable changes in gene function that occur without a change in the genomic DNA sequence. In addition to RNA-associated silencing and histone modification, a major epigenetic mechanism in higher eukaryotes is DNA methylation. DNA methylation in eukaryotes occurs predominantly on cytosine residues, especially on CpG dinucleotides enriched in small DNA regions that are known as CpG islands. These regions are usually clustered around the regulatory regions of genes and can affect the transcriptional regulation of these genes.

Methylation of CpG islands by DNA methylases has been shown to be associated with gene inactivation and plays an important role in the development of cancer and cell aging. Reversal of DNA methylation at these sites has been discussed as a potential therapeutic strategy as this reversal may restore expression of transcriptionally silenced genes.

The most abundant form of DNA methylation is 5-methylated cytosine (5-mC). A decade ago, it was discovered that part of the 5-mC in the genome is converted to 5-hydroxymethylated cytosine (5-hmC) by the Ten-eleven translocation (TET) enzyme family. 5-hmC, an oxidized form of 5-mC, has been proposed for use as a prognostic marker for diagnosing different forms of cancers, in particular prostate cancer. Accordingly, there is a need for methods that can reliably detect 5-hmC.

The methylation status of a DNA sequence is commonly determined by use of bisulfite salts, such as ammonium bisulfite or sodium bisulfite. Incubation of the target DNA with bisulfite salts results in conversion of unmethylated cytosine residues into uracil, leaving the methylated cytosines unchanged. Therefore, bisulfite treatment gives rise to different DNA sequences for methylated and unmethylated DNA. Kits for analyzing the methylation status of DNA via bisulfite treatment are commercially available.

While bisulfite treatment allows for the discrimination between methylated and unmethylated DNA, it is not possible to distinguish between 5-mC and 5-hmC residues. For this reason, it has been suggested in US 9,290,807 to combine bisulfite treatment with a preceding oxidation step that is able to convert 5-hmC, but not 5-mC, into 5-formylcytosine (5-fC) which is then reacted to uracil during bisulfite treatment. By combining bisulfite treatment with a preceding oxidation step, one can distinguish between 5-mC and 5-hmC, as only the latter is converted to uracil in the bisulfite reaction. Thus, by conducting bisulfite treatment with or without oxidation in parallel, one can deduce the C, 5-mC, and 5-hmC residues in a given DNA sequence from the uracil pattern determined by DNA sequencing.

Despite the progress that has been made in this field, the determination of modified and unmodified cytosine residues in nucleic acid sequences is still cumbersome and there is a clear need for simplified processes. The present invention satisfies this need and provides other advantages as well.

### DESCRIPTION OF THE INVENTION

The present invention is based on the insight that commonly available methods for determining 5-mC and 5-hmC residues in a target nucleic acid can be significantly improved by modifying the final detection step. In particular, it has been found that the identification of uracil residues resulting from bisulfite treatment can be achieved easier and quicker by amplification-based methods.

Accordingly, in a first aspect, the invention provides a method of detecting 5-hydroxymethylated cytosine (5-hmC) in a nucleic acid molecule, said method comprising:
(a) providing a sample comprising a nucleic acid molecule;
(b) in a first portion of said sample: oxidizing the nucleic acid molecule and subsequently treating the oxidized nucleic acid molecule with bisulfite;
(c) in a second portion of said sample: treating the non-oxidized nucleic acid molecule with bisulfite;
(d) amplifying the bisulfite-treated nucleic acid molecules obtained from step (b) and step (c) in parallel amplification reactions using at least one oligonucleotide that modulates the reactions efficiency of said amplification reaction depending on the presence of cytosine or uracil in at least one position of said nucleic acid molecule; and
(e) comparing the amplification efficiencies of the parallel amplification reactions, thereby detecting 5-hmC in the nucleic acid molecule.

In a first step of the method of the invention, a sample comprising a nucleic acid molecule is provided. Preferably, the sample will be a liquid sample in which the nucleic acid to be subjected to the method of the invention occurs in dissolved form. The nucleic acid can be, for example, double-stranded or single-stranded DNA or RNA. It is, however, preferred that the nucleic acid is DNA, and in particular genomic DNA. The nucleic acid can be derived from a mammalian or non-mammalian subject. Preferably, it will be derived from a mammalian subject, and more preferably from a human subject. It can be obtained from a cell or tissue sample of the individual to be tested, such as from biopsy material, skin cells or body fluids. For example, the nucleic acid can be derived from a blood sample of the subject to be tested.

The sample containing the nucleic acid will be divided in two portions. Preferably, these portions will have an equal volume. In step (b) of the method, the first portion of said sample will be subjected to oxidation and subsequent bisulfite treatment. This means that all nucleic acid molecules contained in the first portion of the sample are oxidized and subsequently treated with bisulfite. During the oxidation step, any 5-hmC residue that occurs in the nucleic acid molecule will be converted into 5-fC. Oxidation can be achieved by the addition of oxidizing enzymes or chemicals. Preferably, oxidation is achieved by the addition of one or more oxidizing agents. As used herein, the term "bisulfite" refers to an sulphur-oxygen compound in which sulphur is present with an oxidation number of +4. Accordingly, the term comprises e.g. sulphite, hydrogensulfite, and disulfite. Preferred bisulfite compounds include ammonium bisulfite and sodium bisulfite.

The oxidizing agent will be selected such that degradation of the nucleic acid to be treated is minimized. At the same time, the oxidizing agent should not interfere with the subsequent bisulfite reaction. Suitable oxidizing agents, which can be used in the context with the method of the present invention are well known in the prior art and which have been described in the context with bisulfite treatment, include metal oxides, in particular KRuO₄, MnO₂ and KMnO₄. In a preferred embodiment, the oxidizing agent is a perruthenate (RuO₄⁻), such as a perruthenate selected from the group consisting of sodium perruthenate (NaRuO₄), potassium perruthenate (KaRuO₄), tetraalkylammonium perruthenate, tetrapropylammonium perruthenate and tetrabutylammonium perruthenate.

For oxidizing the nucleic acid, the nucleic acid is preferably denatured and subsequently incubated in the presence of the oxidizing agent, preferably at a temperature between 25-40°C, such as 37°C. If necessary, the oxidation can be repeated one or several times to ensure that all 5-hmC residues are completely converted. Following oxidation, the nucleic acid may be purified using routine techniques and kits, such as the QIAquick PCR Purification kit or the Merck CpGenome Turbo Bisulfite Modification Kit.

After the oxidation step has been completed, and the oxidized nucleic acid molecule has been optionally purified, the oxidized nucleic acid molecule present in the first portion of the sample is treated with bisulfite. Bisulfite treatment can be effected by use of a commercial kit like the Qiagen EpiTect^{®} Bisulfite kit according to the manufacturer's instructions. The bisulfite will convert both unmethylated cytosine residues and 5-fC residues in the nucleic acid into uracil residues.

In a parallel approach, the nucleic acid molecules in the second portion of the nucleic acid sample are also treated with bisulfite in step (c) of the above method. Unlike the nucleic acid molecules in the first portion of the sample, the nucleic acid molecules in the second portion of the sample have not been oxidized prior to bisulfite treatment.

In step (d) of the above method, the bisulfite-treated nucleic acid molecules obtained from step (b) and step (c) are amplified in parallel amplification reactions. In this step, at least one oligonucleotide is used that modulates the amplification efficiency depending on the presence of cytosine or uracil in at least one position of the nucleic acid molecule. As used herein, the term oligonucleotide refers to a polynucleotide consisting of at least 5 nucleotides, preferably at least 10, 15, 20, 25, 30 or 35 nucleotides. The length of the oligonucleotide can be up to 35 nucleotides, preferably up to 40, 45, 50, 55, or 60 nucleotides. Stated differently, an oligonucleotide as defined herein can have a length of 5-60, more preferably 10-50, 15-40, 20-35 or 25-35 nucleotides. They can be prepared by any suitable technique known in the art. The oligonucleotides for use in the methods of the present invention may also contain modified bases, such as 2-thiouracil, 8-thiolguanine, 7-methylguanine, 7-methyladenine, 8-azaguanine und 8-azaadenine, 7-deazaguanine, 7-deazaadenine, 3-deazaguanine and/or 3-deazaadenine. The oligonucleotides for use in the methods of the present invention may also contain Locked Nucleic Acids (LNAs) and other modified bases which increase the melting temperature (Tₘ).

In a particular preferred embodiment, the oligonucleotide is a primer that modulates the efficiency of the amplification reaction depending on whether a cytosine or uracil occurs in at least one position within the binding region of the primer in the target nucleic acid molecule. This means that the oligonucleotide primer can be designed such that it exerts enhanced binding to the bisulfite-treated nucleic acid molecules obtained from step (b) and step (c) if said molecules contain at least one uracil residue in a pre-determined position. Alternatively, the oligonucleotide primer can be designed such that it exerts enhanced binding to the bisulfite-treated nucleic acid molecules obtained from step (b) and step (c) if said molecules contain at least one unmodified cytosine residue in a pre-determined position. The pre-determined position can be located within the binding region of the primer. For example, it can be at the 3' end of the primer or in the middle of the primer (e.g. flanked by at least 5-10 nucleotides to both ends). However, it is preferred that the pre-determined position is located essentially in the middle of the primer.

For example, as explained in the below example, the primer can be designed such that a G residue is located at its 3' end which is supposed to bind to a corresponding C residue in the bisulfite-treated nucleic acid. If the bisulfite-treated nucleic acid contains a C residue in this position, the primer will perfectly match with the target. If the position in the bisulfite-treated nucleic acid that corresponds to the 3' end of the primer however is a U residue, this mismatch will reduce the extent of primer binding, and the amplification efficiency resulting from a reaction with such a primer will be lower compared to the perfectly matching primer. On the other hand, the primer can be designed such that an A residue is located at its 3' end which is supposed to bind to a corresponding U residue in the bisulfite-treated nucleic acid. If the bisulfite-treated nucleic acid contains a U residue in this position, the primer will perfectly match with the target. If the position in the bisulfite-treated nucleic acid that corresponds to the 3' end of the primer however is a C residue, this mismatch will reduce the extent of primer binding, and the amplification efficiency resulting from a reaction with such a primer will be lower compared to the perfectly matching primer. In this way, the use of primer pairs that are adapted to either C or U residues in the target nucleic acid can be used for discriminating between these nucleotides and thereby allow to conclude whether 5-hmC residues occurred in a particular position of the target nucleic acid.

For example, an uracil residue at a position in the bisulfite-treated nucleic acid molecule obtained from step (b) of the above method which corresponds to a cytosine in the bisulfite-treated nucleic acid molecule obtained from step (c) of the above method indicates that 5-hmC is present at this position in the nucleic acid molecule that was initially provided in step (a) of the method.

According to the present invention, it is also possible to use an oligonucleotide in step (d) that modulates the signal intensity of a probe. Accordingly, the present invention also provides a method of detecting 5-hydroxymethylated cytosine (5-hmC) in a nucleic acid molecule, said method comprising:
(a) providing a sample comprising a nucleic acid molecule;
(b) in a first portion of said sample: oxidizing the nucleic acid molecule and subsequently treating the oxidized nucleic acid molecule with bisulfite;
(c) in a second portion of said sample: treating the non-oxidized nucleic acid molecule with bisulfite;
(d) amplifying the bisulfite-treated nucleic acid molecules obtained from step (a) and step (b) in parallel amplification reactions using at least one oligonucleotide that modulates the signal intensity of a probe depending on the presence of cytosine or uracil in at least one position of said nucleic acid molecule; and
(e) comparing the signal intensities of the probe as detected in the parallel amplification reactions, thereby detecting 5-hmC in the nucleic acid molecule.

In such an embodiment, the oligonucleotide modulates the signal intensity of a probe depending on the presence of cytosine or uracil in at least one position of said nucleic acid molecule. This means that the oligonucleotide itself can be a probe that binds to the nucleic acid molecule dependent on whether there is a C or U residue in one or more positions of the nucleic acid molecule to which it binds. In that case, the probe can be designed as explained above in the context with primers, i.e. the probe either exerts enhanced binding to the bisulfite-treated nucleic acid molecules if said molecules contain at least one unmodified cytosine residue, or, alternatively, the probe exerts enhanced binding to the bisulfite-treated nucleic acid molecules if said molecules contain at least one uracil residue in a pre-determined position. By selecting stringent conditions, different signal intensities will be observed. The pre-determined position can be located within the binding region of the probe. For example, it can be at the 3' end of the probe or in the middle of the probe (e.g. flanked by at least 5-10 nucleotides to both ends). However, it is preferred that the pre-determined position is located essentially in the middle of the probe.

The oligonucleotide may also be a blocking oligonucleotide which competes with a primer or probe for the binding site in the target nucleic acid. In such a case, a perfectly matching blocking oligonucleotide will provide for a better binding to the target site than a probe that contains at least one mismatch. Since the blocking oligonucleotide competes with the labeled probe for the binding site within the nucleic acid molecule, a reduced signal intensity can be observed if the blocking oligonucleotide, but not the labeled probe, is completely complementary to the target site within the nucleic acid molecule.

Based on the present disclosure, a skilled person will be readily able to design suitable primers and probes for use in amplification reactions that allow the determination of a C or U residue in a certain position within the nucleic acid molecule. High amplification efficiencies can be determined, for example, by measuring the ct-values. A low ct-value is associated with high amplification efficiencies.

In step (d) of the above methods, the primers and probes described elsewhere herein are used in an amplification reaction in order to multiply the bisulfite-treated nucleic acid molecules. In a simple embodiment, a polymerase chain reaction (PCR) is used to amplify the bisulfite-treated nucleic acids. A PCR is an enzymatic reaction for increasing the copy number of DNAs molecule which is catalyzed by a thermostable DNA-dependent DNA polymerase. The primers are complementary to one strand of the double-stranded target sequence. For amplification, the double-stranded target sequence is denatured so as to allow annealing of the primers. Following annealing, the primers are extended by means of the DNA polymerase to form a new pair of complementary strands. The steps of denaturation, primer annealing, and polymerase extension are repeated several times to multiply the template DNA. PCR amplification methods are well-known to the skilled person, and PCR kits are commercially available from many different manufacturers.

In a more preferred aspect, the probes and primers described elsewhere herein are amplified by real-time PCR. In a real-time PCR method, it is possible to monitor the multiplication of the PCR products and quantify the amplified products in real time during amplification. The method is based on the principle that a detectable emission of fluorescence from dyes is directly or indirectly associated with the formation of a newly-synthesized amplification product in each PCR cycle. Commonly known real-time PCR methods can use different types of probes, such as TaqMan probes, molecular beacons, scorpion probes, iFRET probes, and the like. These probes are based on the "fluorescence resonance energy transfer" (FRET) technique and rely on the principle that a donor fluorophore absorbs excitation energy and delivers this energy to an acceptor fluorophore (also referred to as quencher) when the donor and acceptor molecules are sufficiently close to one another. When the donor fluorophore and the acceptor fluorophore are separated in the amplification reaction, the increased distance between the fluorophores leads to an increased fluorescence signal of the donor fluorophore and a decreased signal of the acceptor fluorophore owing to the decreased level of fluorescence resonance energy transfer. An alternative to probe-based methods is the use of DNA intercalating dyes that bind to double-stranded DNA. Usable dyes have much higher fluorescence when bound to double-stranded DNA compared to the unbound state. Indication of specific amplification is subsequently obtained by analysis of the melting curve of the PCR amplicons.

In one embodiment, the oligonucleotide used in step (d) of the above methods is a FRET probe for use in a real-time PCR method. In a preferred embodiment, the probe is a molecular beacon. As used herein, a molecular beacon is a single-stranded oligonucleotide having a stem-loop structure, i.e. the probe forms a hairpin structure in which the donor fluorophore and the acceptor fluorophore are located at opposite ends of the oligonucleotide. Owing to the stem-loop structure, donor fluorophore and acceptor fluorophore are in close proximity with each other. The loop portion of the molecular beacon will be complementary to the target nucleic acid, i.e. the nucleic acid obtained after bisulfite treatment. When the molecular beacon binds to the target nucleic acid, the stem structure is destroyed and the distance between donor and acceptor fluorophores is increased. Methods for the production of molecular beacons are extensively described in the scientific literature.

In another preferred embodiment, the probe is a TaqMan probe. A TaqMan probe is a single-stranded oligonucleotide that is complementary to a segment of 10-50 nucleotides within the target nucleic acid located between the two PCR primers. A fluorophore and a quencher dye are covalently attached to the 5' and 3' ends of the probe, respectively. Alternatively, the quencher dye can also be attached to an internal nucleotide, while the fluorophore is attached to the 5' or 3' end of the probe or vice versa. The close proximity between fluorophore and quencher dye attached to the probe inhibits fluorescence emission from the fluorophore when said fluorophore is selectively excited. During DNA synthesis in the PCR reaction, the 5' exonuclease activity of the Taq polymerase cleaves the oligonucleotide probe which is hybridized to the template DNA, thereby spatially separating the fluorophore and the quencher dye. The fluorescence detected during PCR cycling is directly proportional to the fluorophore released and the amount of DNA template in the PCR reaction. Each fluorophore-quencher pair known in the art can be used in the methods of the present invention. Examples of suitable fluorophores are FAM (6-carboxyfluorescin), TET (tetrachlorofluorescin) or VIC (2'-chloro-7'-phenyl-1,4-dichloro-6-carboxy-fluorescein). A suitable quencher dye is TAMRA (tetramethylrhodamine).

The construction and labelling of the oligonucleotide to be used in a TaqMan approach have been extensively described in the scientific literature. It is also possible to use differently labeled probes in the same TaqMan assay. For example, one TaqMan probe being specific for a bisulfite-treated target nucleic acid containing one or more uracil residues can be labeled with FAM, while another TaqMan probe being specific for the corresponding bisulfite-treated target nucleic acid containing one or more unmodified cytosine residues can be labeled with VIC. In such a method, a FAM fluorescence which is higher than VIC fluorescence would indicate the presence of uracil in the target nucleic acid. Conversely, a VIC fluorescence which is higher than FAM fluorescence would indicate the presence of unmodified cytosine residues in the corresponding positions of the target nucleic acid. All probes used in the methods of the present invention may contain chemical modifications or modified nucleotides which modulate the melting temperature (Tₘ). Such modifications preferably increase the melting temperature of the probe, such as nucleotides that include a or more minor groove binders (MGBs).

The present invention also relates to kits for performing the methods of the present invention. In one embodiment, a kit for detecting 5-hydroxymethylated cytosine (5-hmC) in a nucleic acid molecule is provided, said kit comprising
(a) an oxidizing agent for oxidizing a nucleic acid molecule;
(b) a bisulfite compound for converting cytosines in a nucleic acid molecule into uracil;
(c) a first and second set of oligonucleotide primers for amplifying a target nucleic acid sequence, wherein the first primer set is adapted to provide a higher amplification efficiency than the second primer set due to the presence of uracil in at least one position of said target nucleic acid sequence.

In one embodiment, the oxidizing agent contained in the kit is a perruthenate (RuO₄⁻) compound, such as NaRuO₄ or KRuO₄. In another embodiment, said bisulfite compound contained in the kit is a bisulfite salt. Preferably, the bisulfite salt is ammonium bisulfite or sodium bisulfite. In yet another embodiment, the kit further comprises, as component (d), PCR reagents, in particular buffers, diluents and enzymes, such as polymerases which are useful for amplifying nucleic acid molecules. Alternatively or in addition thereto, the kit may comprise suitable probes which are labeled, e.g. probes for TaqMan PCR or hybridization probes. The kit will further comprise instructions for using the components in a method for determining 5-hmC residues in nucleic acid molecule.

The oligonucleotide primers contained in the kit are designed to allow the discrimination between nucleic acid molecules which differ from each other merely by the presence of one or more uracil residues instead of cytosine residues.

### BRIEF DESCRIPTION OF THE FIGURES

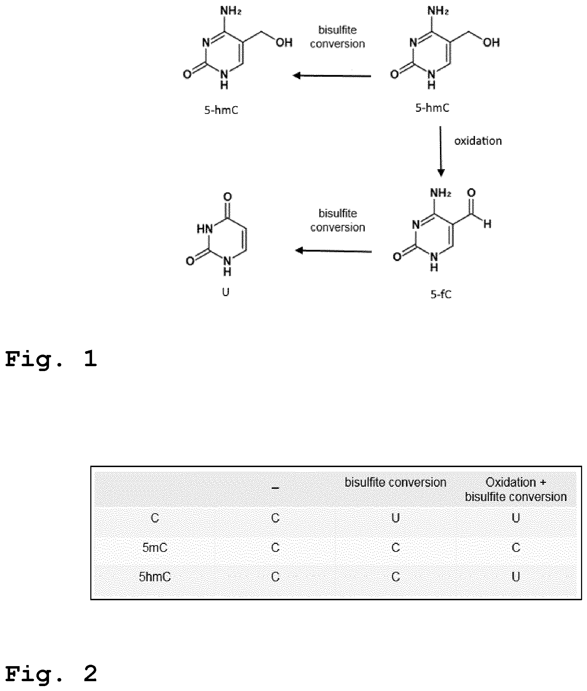

Fig. 1 shows the reaction scheme of the conversion of 5-hmC to 5-fC and U after oxidation and bisulfite conversion.
Fig. 2 shows the conversion of C, 5mC and 5-hmC residues after bisulfite conversion with or without previous oxidation.
Fig. 3 shows the sequence of the DNA amplified in Example 1. The position of the PCR primers is framed. Primer positions critical for PCR detection of modified nucleotides are indicated with an arrow.

### EXAMPLES

The present invention will be described in the following by preferred embodiments which merely illustrate the invention, but should by no means limit the invention.

### Example 1: Preparation of DNA templates

For the subsequent analysis, PCR fragments with modified and non-modified cytosine residues were amplified from a genomic DNA template isolated from human Jurkat cells using the QIAGEN HotStar Taq PCR Kit (QIAGEN, Hilden, Germany) as described in the corresponding handbook.

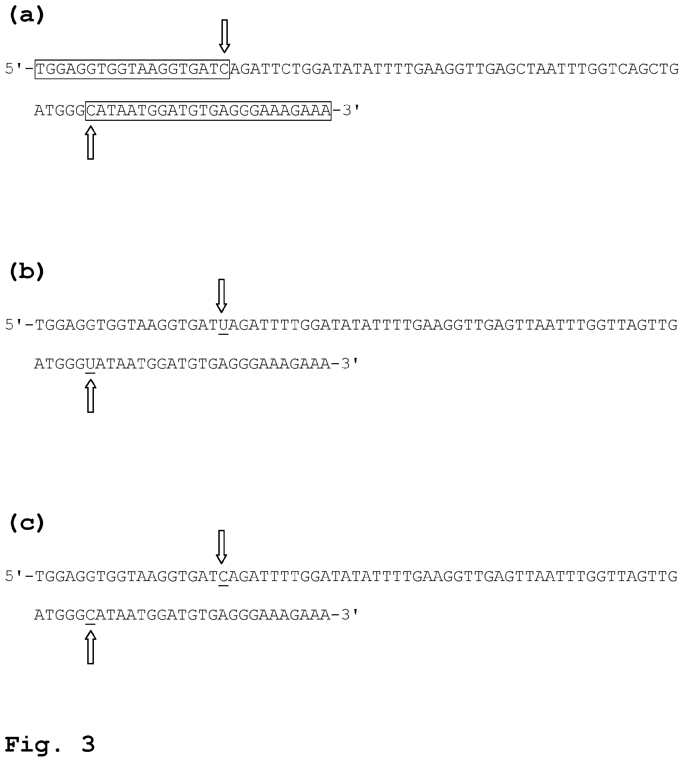

The sequence of the template is depicted in Figure 3(a) and set out in the attached sequence listing as SEQ ID N0:1. The cytosine residues occurring in the template are indicated with arrows in Figure 3(a). The following primers were used in the amplification process:
F1-C (SEQ ID NO:2)
   5'-GTGGAGGTGGTAAGGTGATC-3'
R1-G (SEQ ID NO:3)
   5'-TTTCTTTCCCTCACATCCATTATG-3'

The binding position of the primers in the template sequence is indicated by frames in Figure 3(a). In the amplification process, the following different dCTPs were used:
- dCTP:: deoxycytosine triphosphate, the unmodified "standard" dCTP
- 5m-dCTP: deoxy-5-methylcytosine triphosphate, a dCTP methylated at position 5
- 5hm-dCTP: deoxy-5-hydroxymethylcytosine triphosphate, a dCTP hydroxymethylated at position 5

As a result, the resulting amplicons were either unmodified (amplicon I, "C"), fully methylated (amplicon II, "5-mC") or fully hydroxymethylated (amplicon III, "5-hmC") at any occurring cytosine positions. After amplification, the amplicons were purified by use of the QIAquick PCR clean-up Kit (QIAGEN, Hilden, Germany) according to the instructions provided in the corresponding handbook.

### Example 2: Bisulfite conversion

The purified DNA amplicons I-III obtained in Example 1 were then subjected to bisulfite treatment.

In a first aliquot of each amplicon obtained in Example 1 ("A"), bisulfite conversion was effected by use of the EpiTect Fast Bisulfite Kit (QIAGEN, Hilden, Germany) following the instructions provided in the corresponding handbook. In this aliquot, any unmodified C residues occurring in the amplicon are converted into U residues (Figure 3(b)), whereas the methylated and hydroxymethylated 5-mC and 5-hmC residues remain unchanged (Figure 3(c)).

In a second aliquot of each amplicon obtained in Example 1 ("B"), the nucleic acids were first oxidized by using potassium perruthenate as described by Booth, et al (2012). Briefly, 100 ng DNA were denatured in 0.1 M NaOH (final concentration) for 10 min at 37°C in a final volume of 19 µl. Subsequently, 1 µl of 50 mM KRuO₄ was added, and the mixture was incubated for 1 h at 37°C (Thermomixer, 350 rpm). After the addition of 4 µl of 0.2 M NaOH the mixture was incubated for 10 min at 37°C. Finally, 1 µl of 50 mM KRuO₄ was added, and the mixture was incubated again for 1 h at 37°C (Thermomixer, 350 rpm). In this aliquot, both the unmodified C residues and 5-hmC residues occurring in the amplicon are converted into U residues (Figure 3(b)), whereas 5-mC residues are not modified (Figure 3(c)).

Accordingly, if a certain position in the template DNA is detected as a "C" after bisulfite treatment, but gives rise to an "U" after oxidation and subsequent bisulfite treatment, it can be concluded that a 5-hmC residue occurs at this position. By examining the template DNA in parallel batches that have been treated by (i) bisulfite treatment and (ii) oxidation and subsequent bisulfite treatment, one can reliably analyze the presence of 5-hmC residues in a nucleic acid molecule.

### Example 3: Detection of 5-hmC in the DNA template

For detecting the presence of uracil or cytosine in the DNA amplicons from aliquots (A) and (B), quantitative real-time PCR analysis was performed with the QuantiFast SYBRGreen PCR Kit (QIAGEN, Hilden, Germany) according to the manufacturer's recommendations. The differentiation between unmodified cytosine and uracil residues was achieved by use of primers with either matching or non-matching 3'-ends.

As primers with matching ends, the primers described above in connection with Example 1 were used:
F1-C (SEQ ID NO:2)
   5'-GTGGAGGTGGTAAGGTGATC-3'
R1-G (SEQ ID NO:3)
   5'-TTTCTTTCCCTCACATCCATTATG-3'

These primers, which are also referred to as the "C/G primer pair" in the following, perfectly match to the original sequence giving low ct-values and high amplification efficiency regardless of the modification of the cytosines (non-modified, methylated, or hydroxy methylated).

As primers with non-matching ends, the following primers were used:
F1-T
   5'-GTGGAGGTGGTAAGGTGATT-3'
R1-A
   5'-TTTCTTTCCCTCACATCCATTATA-3'

These primers, which are also referred to as the "A/T primer pair" in the following, match to uracil residues after conversion of the cytosine into uracil in the bisulfite reaction (like thymine, uracil binds to adenine during base pairing).

When both primer pairs are used in parallel amplification reactions with the same amplicon, it is possible to discriminate between "C" and "U" residues by comparing the relative ct-values. By such comparison it possible to identify 5-hmC residues in the original nucleic acid molecule.

The following Tables 1 & 2 show the results from measuring the ct-values for each of amplicon I ("C"), amplicon II ("5-mC"), and amplicon III ("5-hmC") as obtained from Example 1 when used in quantitative real-time PCR with the A/T primer pair and the C/G primer pair. Table 1 shows the results after bisulfite treatment, whereas Table 2 depicts the results after oxidation and subsequent bisulfite treatment.

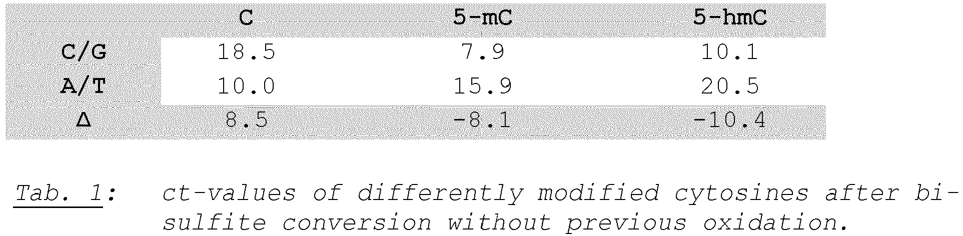

It can be seen in Table 1 that in the amplicon containing unmodified cytosines (amplicon "C"), the ct values measured with the A/T primer pair is significantly lower compared to the C/G primer pair which indicates that the amplicon contains uracil residues which perfectly match to the A/T primer pair, but not with the C/G primer pair. For the other two amplicons containing the modified cytosines (amplicons "5-mC" and "5-hmC"), the situation is different. In these amplicons, bisulfite treatment did not result in the conversion of cytosines to uracils. Accordingly, these amplicons exert lower ct values with the C/G primer pair.

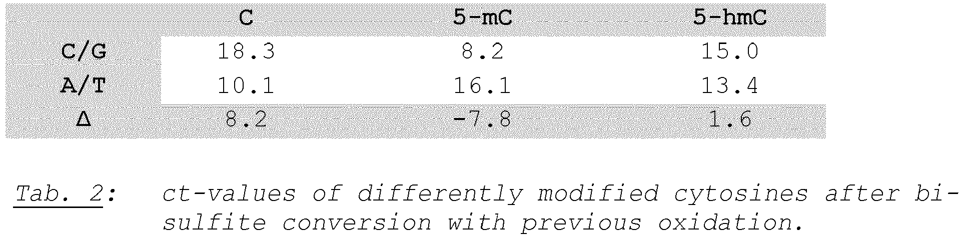

The data contained in Table 2 demonstrate that for the amplicon containing the unmodified cytosines (amplicon "C"), essentially the same ct values were measured for both primer pairs which is not surprising when considering that oxidizing the DNA prior to bisulfite treatment, just like bisulfite treatment alone, leads to a conversion to uracil. The ct values measured for amplicon "5-mC" are likewise comparable to those depicted in Table 1. Again, 5-mC is modified neither by bisulfite treatment nor by oxidation and bisulfite treatment. For amplicon "5-hmC", however, there is a clear difference between Table 1 and Table 2. While the ct value after bisulfite treatment is lower for the C/G primer pair, said value is lower for the A/T primer pair after oxidation and bisulfite treatment.

All results obtained with the PCR primers were confirmed by DNA sequencing.

## Claims

1. A method of detecting 5-hydroxymethylated cytosine (5-hmC) in a nucleic acid molecule, said method comprising:
(a) providing a sample comprising a nucleic acid molecule;
(b) in a first portion of said sample: oxidizing the nucleic acid molecule and subsequently treating the oxidized nucleic acid molecule with bisulfite;
(c) in a second portion of said sample: treating the non-oxidized nucleic acid molecule with bisulfite;
(d) amplifying the bisulfite-treated nucleic acid molecules obtained from step (a) and step (b) in parallel amplification reactions using at least one oligonucleotide that modulates the amplification efficiency depending on the presence of cytosine or uracil in at least one position of said nucleic acid molecule; and
(e) comparing the amplification efficiencies of the parallel amplification reactions, thereby detecting 5-hmC in the nucleic acid molecule.

2. Method according to claim 1, wherein said nucleic acid molecule is a DNA molecule, preferably a genomic DNA molecule.

3. Method according to any of claims 1-2, wherein said first portion of said sample is oxidized in step (b) with a perruthenate (RuO₄⁻) compound.

4. Method according to claim 3, wherein said perruthenate (RuO₄⁻) compound is NaRuO₄ or KRuO₄.

5. Method according to any of claims 1-4, wherein said bisulfite used in steps (b) and (c) is sodium bisulfite or ammonium bisulfite.

6. Method according to any of claims 1-5, wherein said amplification reactions in step (d) comprise PCR, preferably real-time PCR.

7. Method according to any of claims 1-6, wherein said at least one oligonucleotide that modulates the amplification efficiency is a PCR primer.

8. Method according to any of claims 1-6, wherein said at least one oligonucleotide that modulates the amplification efficiency is a PCR probe.

9. Method according to claim 8, wherein said PCR probe is a FRET, a molecular beacon or a TaqMan probe.

10. Kit for detecting 5-hydroxymethylated cytosine (5-hmC) in a nucleic acid molecule, said kit comprising
(a) an oxidizing agent for oxidizing a nucleic acid molecule;
(b) a bisulfite compound for converting cytosines in a nucleic acid molecule into uracil;
(c) optionally a first and second set of oligonucleotide primers for amplifying a target nucleic acid sequence, wherein the first primer set is adapted to provide a higher amplification efficiency than the second primer set due to the presence of uracil in at least one position of said target nucleic acid sequence.

11. Kit according to claim 10, wherein said oxidizing agent is a perruthenate (RuO₄⁻) compound.

12. Kit according to claim 11, wherein said perruthenate (RuO₄⁻) compound is NaRuO₄ or KRuO₄.

13. Kit according to any of claims 10-12, wherein said bisulfite compound is sodium bisulfite or ammonium bisulfite.

14. Kit according to any of claims 10-13, wherein the kit further comprises (d) reagents for performing a PCR, such as buffers, diluents or enzymes.

15. Method according to any of claims 10-14, wherein the kit further comprises a polymerase enzyme.
